# DEMANDE DE BREVET EUROPEEN

(11) **EP 4 458 346 A1**
(43) Date de publication de la demande: **06.11.2024**
(21) Numéro de dépôt: 24172760.1
(22) Date de dépôt: 26.04.2024
(51) Int. Cl.: A61K 8/9789, A61P 17/08, A61Q 19/00

(54) **EXTRAITS ENRICHIS EN OXYLIPINES, ISSUS DE SILYBUM MARIANUM (L.) GAERTN**

(30) Priorité: 04.05.2023 FR 2304485
(71) Demandeur: Pierre Fabre Dermo-Cosmétique, 81500 Lavaur (FR)
(72) Inventeur: LETI, Mathieu, 81106 CASTRES (FR); DUNOUAU, Christophe, 81106 CASTRES (FR); GARIDOU, Lucile, 81106 CASTRES (FR); MIAS, Céline, 81106 CASTRES (FR); SAURAT, Jean-Hilaire, 1206 GENEVE (CH)
(74) Mandataire: Regimbeau

(57) **Abrégé**

La présente invention concerne un extrait enrichi en oxylipines d'akènes de *Silybum marianum* (L.) Gaertn. pour son utilisation dans le traitement de la séborrhée.

La présente invention concerne également une composition dermo-cosmétique ou dermatologique comprenant un extrait enrichi en oxylipines d'akènes de Silybum marianum (L.) Gaertn. avec au moins un excipient dermo-cosmétiquement ou dermatologiquement acceptable, pour son utilisation dans le traitement de la séborrhée.

## Description

### Domaine technique de l'invention

La présente invention concerne des extraits enrichis en oxylipines, issus de *Silybum marianum* (L.) Gaertn. ou des compositions cosmétiques ou dermatologiques comprenant de tels extraits, pour leur utilisation dans la séborégulation, et en particulier pour le traitement de la séborrhée.

### Etat de la technique

Les organismes vivants sont formés par une ou plusieurs cellules, procaryotes ou eucaryotes, dont les fonctions de survie, de défense et de prolifération reposent sur le métabolisme de trois grandes familles chimiques que sont les glucides, les protides et les lipides.

Les lipides sont des substances naturelles, leurs rôles sont fondamentaux. Ils sont des constituants majeurs des structures cellulaires, tels que les phospho- et glyco-lipides membranaires. Ce sont également des substances de réserve, en tant que source d'énergie pour l'organisme. Ils sont impliqués dans des mécanismes de défense ou de signalisation cellulaire et constituent des éléments de revêtement comme les cires ou les cutines (Cuvelier et al., 2004).

Les lipides sont des substances hydrophobes et parfois amphiphiles, solubles dans les solvants organiques apolaires ou moyennement polaires.

On peut distinguer les lipides simples, esters d'acides gras et d'un alcool qui peut être le glycérol (constitutif des triglycérides) ou un alcool aliphatique de masse moléculaire élevée (constitutif des cérides), des lipides complexes tels que les phospholipides ou glycolipides.

Les acides gras sont des éléments constitutifs élémentaires des lipides. Ils peuvent avoir deux origines additives dans un organisme : la synthèse par l'organisme et l'alimentation.

Les acides gras sont des acides aliphatiques mono-carboxyliques de longueur variable. Les acides gras naturels les plus communs possèdent entre 4 et 28 atomes de carbone. La longueur de leur chaine carbonée, le nombre et la nature de leur(s) insaturation(s) sont également variables. On distingue en effet, les acides gras saturés, monoinsaturés (une seule double liaison) et polyinsaturés (plusieurs doubles liaisons). Les acides gras insaturés peuvent contenir 1 à 6 doubles liaisons dans le règne vivant. Pour une même formule chimique, il peut exister un grand nombre d'isomères, en fonction de la position de la double liaison mais aussi en fonction de sa conformation cis ou trans.

Dans le règne végétal, l'acide gras peut être oxydé sous forme cétonique (e.g. acide licanique), hydroxylé (e.g. acide ricinoléique) ou sous forme époxy (e.g. acide vernolique).

En général, les triglycérides sont hétérogènes et une huile végétale est un mélange complexe de triesters.

Dans le monde végétal, les triacylglycérols sont généralement stockés sous forme d'inclusions huileuses (des oléosomes issus du réticulum endoplasmique) qui, parfois, confluent en gros amas dans les cellules des tissus de réserve. Cela est particulièrement vrai au niveau des graines dans lesquelles ils peuvent représenter plus de 50% de la masse sèche. Exceptionnellement, la graine peut accumuler non pas des triglycérides, mais des esters d'acides gras et d'alcools aliphatiques à longue chaine (e.g. jojoba). Bien que cela soit moins fréquent, il existe des fruits qui concentrent les triglycérides dans leur péricarpe (olive, avocat, baie de laurier...).

Les Oméga 3 et Oméga 6, importants chez l'homme, tels que l'acide arachidonique (C20:4 n-6), l'acide eicosapentaénoïque (C20:5 n-3) et l'acide docosahexaénoïque (C22:6 n-3) ne peuvent être synthétisés par l'arsenal enzymatique des vertébrés sans l'apport indispensable d'acide linoléique et d'acide alpha-linolénique. Ces nutriments sont par conséquents dits essentiels et doivent obligatoirement être apportés par l'alimentation. Ces acides gras polyinsaturés sont majoritairement présents dans l'alimentation sous forme de triacylglycérols, d'esters de cholestérol, de phospholipides, estérifiés ou non, ou sous forme libre (Liu et al., 2015).

Les acides gras polyinsaturés sont le substrat de nombreuses réactions enzymatiques ou non enzymatiques à l'origine de métabolites oxygénés aux fonctions diverses appelés oxylipines. Les oxylipines comprennent les acides gras polyinsaturés oxydés et leurs dérivés. Ils sont présents chez les mammifères, les plantes à fleurs (angiospermes), les mousses, les algues, les bactéries et les champignons. Parmi les oxylipines, on compte des molécules de signalisation, des ligands aux facteurs de transcription, ou des précurseurs de la synthèse de médiateurs lipidiques (Calder, 2012). Par exemple, la classe des eicosanoïdes, parmi lesquels on compte les leucotriènes et les prostanoïdes, constitue une vaste famille de dérivés d'oxydation d'acides gras polyinsaturés à 20 atomes de carbone. Les docosanoïdes sont quant à eux des produits d'oxydation enzymatique d'acides gras polyinsaturés à 22 atomes de carbone tels que l'acide docosahexaénoïque. Les produits d'oxydation des acides gras polyinsaturés à 18 atomes de carbone tels que l'acide linoléique ou l'acide gamma-linolénique appartiennent à la classe des octadécanoïdes. On peut citer parmi les octadécanoïdes issus de l'acide linoléique l'acide 9-hydroxyoctadécadiénoïque (9-HODE) ainsi que l'acide 13-hydroxyoctadécadiénoïque (13-HODE). Ces deux molécules sont décrites comme un marqueur d'oxydation *in vivo* (Lagarde, 2011). Des formes hydroperoxy, époxy, ou cétoniques dérivées de l'acide linoléique sont également documentées (Richardson et al., 2017). Le métabolite oxygéné le plus connu de l'acide gamma-linolénique est celui conduisant à l'acide jasmonique, un dérivé cyclique aux activités bien décrites de résistance aux phyto-pathogènes (Blée, 2002).

L'oxydation des acides gras polyinsaturés peut se faire par voie non enzymatique, ainsi que par voie enzymatique (Oenel et al., 2017). Pour cette dernière, la position des insaturations sur l'acide gras polyinsaturé, ainsi que l'environnement enzymatique définiront le type d'oxylipines formées. Les voies principales sont celles des cyclooxygénases, des lipoxygénases et des cytochrome P-450 (Andreou et al., 2009). Si la voie enzymatique semble être la principale voie de formation d'acides gras oxygénés à partir d'acides gras polyinsaturés sous forme libre, certaines études montrent que l'oxydation d'acides gras polyinsaturés, notamment sous leur forme estérifiée (triglycérides notamment), peut se faire par voie non enzymatique. Celle-ci peut se produire dans la matrice végétale oléagineuse par l'action de radicaux libres dont la formation peut être favorisée par les conditions de stockage (température, durée...). Ces acides gras oxydés peuvent ensuite être libérés après désestérification, par l'action de lipases notamment (Oenel et al., 2017).

Les oxylipines sont présentes dans les huiles végétales polyinsaturées ou les matrices végétales oléagineuses à acides gras polyinsaturés de plantes ou d'algues. Les algues peuvent en effet être des sources d'acides gras polyinsaturés et renferment les enzymes permettant la formation d'oxylipines (Richardson et al., 2017). Leur synthèse peut également être favorisée dans des tissus faiblement oléagineux dans certaines conditions, comme par exemple des feuilles de tabac exposées à la cryptogéine (Rustérucci et al., 1999). Enfin, la demande de brevet FR2789085 décrit un procédé d'obtention d'une huile enrichie en acides gras hydroxyoctadeécadiénoïques et notamment en acide 9-hydroxyoctadécadiénoïque à partir d'un mélange huileux contenant de l'acide linoléique. L'obtention des acides gras hydroxyoctadécadiénoïques se fait par oxydation contrôlée de l'acide linoléique et / ou de l'acide alpha et/ou gamma linolénique ou de leurs esters en présence d'un catalyseur d'oxydation tel que l'halogénure de fer ou de cuivre.

Le nom scientifique *Silybum marianum* (L.) Gaertn. désigne une plante appartenant à la famille des Asteraceae, annuelle ou bisannuelle à tige robuste pouvant atteindre plus d'un mètre de hauteur. Ses grandes feuilles luisantes, alternées, sans stipule sont marbrées de blanc et bordées d'épines dures et pointues. Les fleurs sont groupées en capitules terminaux, souvent solitaires. Ils sont entourés de grandes bractées épineuses à l'extrémité très acérée. Les fleurs, tubulées, à cinq lobes, sont de couleur pourpre violacé. Les fruits sont des akènes luisants, noirs ou marbrés de jaune, surmontés d'une aigrette à soies denticulées en anneau à leur base. Le principal nom vernaculaire de cette plante est Chardon-Marie. Cette plante affectionne particulièrement les lieux secs et ensoleillés, souvent sur sols acides, secs et cailleux. Sa répartition géographique est concentrée sur le pourtour méditerranéen, mais elle est également présente en Europe, en Asie de l'Ouest, ainsi qu'en Amérique du Nord et en Australie ou même en Nouvelle-Zélande. Elle pousse dans les jardins mais elle est plus dominante dans les champs incultes, dans les pâturages, le long des bordures des sentiers et entre les décombres.

L'akène (souvent dénommé graine de manière erronée dans la littérature) de *Silybum marianum* (L.) Gaertn. et ses préparations sont traditionnellement utilisés par voie orale, dans le traitement symptomatique des troubles fonctionnels digestifs attribués à une origine hépatique.

Le principe actif principal de l'akène de *Silybum marianum* (L.) Gaertn. est la silymarine, qui est un mélange de plusieurs flavolignanes. Les akènes contiennent jusqu'à 3% en poids de silymarine. Ils sont également constitués d'huile, de mucilages et de protéines. Les akènes de *Silybum marianum* (L.) Gaertn. contiennent généralement 15 à 30% d'huile. L'huile de *Silybum marianum* (L.) Gaertn. est dépourvue de silymarine ou en contient des traces non détectables. Cela est confirmé par l'analyse de la fraction polyphénols de l'huile de chardon-Marie qui ne révèle en effet la présence d'aucun constituant de la silymarine (Meddeb et al., 2018 ; Zarrouk et al., 2019).

L'huile non raffinée de *Silybum marianum* (L.) Gaertn. est essentiellement composée de triglycérides d'acides gras insaturés dont les majoritaires sont l'acide linoléique (30 à 60%) et l'acide oléique (15 à 30 %). Sa teneur élevée en acides gras insaturés lui permet d'entrer dans les régimes anti-cholestérol et d'être utilisée dans la prévention des maladies cardio-vasculaires (El-Mallah et al., 2003). L'huile contient également des acides gras saturés : acide palmitique (5 à 15%), acide stéarique (3 à 8 %), acide arachidique (1 à 4 %) et acide béhénique (1 à 4 %). L'huile brute obtenue par pression à froid contient également des phytostérols (beta-sitosterol notamment) et des tocophérols (α-tocophérol, et γ-tocophérol notamment) (Dabbour et al., 2014).

L'huile de *Silybum marianum* (L.) Gaertn. est essentiellement utilisée dans le domaine culinaire.

Par ailleurs, des études sur les propriétés antioxydante et hépatoprotectrice de l'huile de *Silybum marianum* (L.) Gaertn., administrée par voie orale, ont été réalisées *in vivo* sur des rats ou des souris (Hermenean et al., 2015 ; Zhu et al., 2014). Plusieurs études *in tubo* ou *in vitro* mettent en évidence les propriétés antioxydante et cytoprotective de l'huile de *Silybum marianum* (L.) Gaertn. obtenue par pression à froid ou par extraction par solvant (Dabbour et al., 2014 ; Harrabi et al., 2018 ; Meddeb et al., 2018).

La glande sébacée est une glande exocrine présente sur l'ensemble de la peau, à l'exception des paumes des mains et des pieds. On la retrouve plus particulièrement dans les régions séborrhéiques du visage, en particulier dans la zone T, c'est-à-dire la zone médiane du visage qui forme un T, du dos et du torse. Elle est généralement associée à un follicule pileux. C'est pourquoi on parle de l'unité pilo-sébacée. La glande sébacée est responsable de la synthèse et de la sécrétion du sébum par les sébocytes, cellules épithéliales qui la composent.

Le sébum est un complexe de lipides synthétisés sous stimulation hormonale. Il s'agit d'un composant essentiel du film hydrolipidique qui protège la peau des agressions extérieures et de la déshydratation en jouant un rôle majeur de barrière. Associé à cette fonction barrière, le sébum a également des propriétés antibactériennes, *via* les acides oléique et palmitoléique et un pouvoir antioxydant notamment apporté par la vitamine E. Ainsi, le sébum aide à maintenir l'intégrité de la barrière cutanée.

La séborrhée correspond à une sécrétion excessive de sébum par les glandes sébacées. Elle occasionne des désagréments d'ordre esthétiques. En ce qui concerne la peau, elle présente un aspect luisant, le teint est terne et les orifices pilo-sébacés sont dilatés. De plus, le maquillage ne présente pas une bonne tenue sur ce type de peaux dites grasses. Pour la séborrhée du cuir chevelu, les cheveux présentent un aspect gras et terne et ils sont difficiles à coiffer. Quand la séborrhée est intense, elle est dite huileuse, fluente et elle peut être associée à une odeur rance.

Ainsi, il existe toujours un besoin de fournir des solutions efficaces pour traiter la séborrhée et résoudre les désagréments d'ordre esthétique liés à la séborrhée.

### Résumé de l'invention

De façon surprenante et inattendue, les inventeurs ont mis en évidence que des extraits enrichis en oxylipines issues d'akènes de *Silybum marianum* (L.) Gaertn. possèdent une activité anti-séborrhéique, et en particulier ont la capacité de diminuer la production de sébum.

La présente invention a ainsi pour objet un extrait enrichi en oxylipines d'akènes de *Silybum marianum* (L.) Gaertn. pour son utilisation dans le traitement de la séborrhée.

L'invention concerne également l'utilisation d'un extrait enrichi en oxylipines d'akènes de *Silybum marianum* (L.) Gaertn. pour la préparation d'une composition dermo-cosmétique ou dermatologique destinée au traitement de la séborrhée.

L'invention concerne également l'utilisation d'un extrait enrichi en oxylipines d'akènes de *Silybum marianum* (L.) Gaertn. pour le traitement de la séborrhée.

L'invention concerne également une méthode de traitement de la séborrhée comprenant l'administration à une personne en ayant besoin d'une quantité efficace d'un extrait enrichi en oxylipines d'akènes de *Silybum marianum* (L.) Gaertn..

La présente invention a également pour objet une composition dermo-cosmétique ou dermatologique comprenant un extrait enrichi en oxylipines d'akènes de *Silybum marianum* (L.) Gaertn. avec au moins un excipient dermo-cosmétiquement ou dermatologiquement acceptable, pour son utilisation dans le traitement de la séborrhée.

L'invention concerne également l'utilisation d'une composition dermo-cosmétique ou dermatologique comprenant un extrait enrichi en oxylipines d'akènes de *Silybum marianum* (L.) Gaertn. avec au moins un excipient dermo-cosmétiquement ou dermatologiquement acceptable, pour la préparation d'un médicament destiné au traitement de la séborrhée.

L'invention concerne également une méthode de traitement de la séborrhée comprenant l'administration à une personne en ayant besoin d'une quantité efficace d'une composition dermo-cosmétique ou dermatologique comprenant un extrait enrichi en oxylipines d'akènes de *Silybum marianum* (L.) Gaertn. avec au moins un excipient dermo-cosmétiquement ou dermatologiquement acceptable.

### Définitions

Par « extrait », on entend, au sens de la présente invention, le produit obtenu après extraction de la plante ou d'une partie de la plante, telle qu'une huile végétale issue de la plante, avec un solvant appelé solvant d'extraction, qui peut ensuite être éventuellement sous une forme concentrée ou sèche après évaporation partielle ou totale du solvant d'extraction. Il peut s'agir en particulier d'un extrait sec.

Par « extrait sec », on entend, au sens de la présente invention, un extrait dépourvu de solvant d'extraction, ou en contenant uniquement à l'état de traces non significative. Un tel extrait sec contient ainsi uniquement de la matière issue de la matière première végétale. Il peut contenir également des traces non significatives de solvant d'extraction.

Par « environ », on entend au sens de la présente invention, que la valeur concernée peut être inférieure ou supérieure de 10%, notamment de 5%, en particulier de 2%, plus particulièrement de 1% à la valeur indiquée.

Par « extrait enrichi en oxylipines », on entend, au sens de la présente invention, un extrait tel que défini ci-dessus obtenu à partir de *Silybum marianum* (L.) Gaertn. ou d'une partie de celle-ci contenant des oxylipines, telle que les achènes, et dont la synthèse d'oxylipines a été stimulée, ou un extrait tel que défini ci-dessus qui a fait l'objet d'une étape d'enrichissement en oxylipines (par ex. par chromatographie).

Par « acide gras », on entend, au sens de la présente invention, un acide carboxylique R1CO₂H dont la chaîne R1 est une chaîne hydrocarbonée linéaire ou ramifiée, saturée ou comprenant une ou plusieurs, notamment 1, 2, 3, 4, 5 ou 6, doubles liaisons C=C, l'acide carboxylique comprenant de 10 à 28, de préférence de 14 à 24, en particulier de 16 à 22, atomes de carbone (incluant l'atome de carbone de la fonction acide carboxylique).

Par « acide gras polyinsaturé », on entend, au sens de la présente invention, un acide gras tel que défini ci-dessus dont la chaîne hydrocarbonée comprenant une ou plusieurs, notamment 1, 2, 3, 4, 5 ou 6, doubles liaisons C=C.

Par « acide gras libre », on entend, au sens de la présente invention, un acide gras non lié à d'autres molécules (par ex. à du glycérol ou des dérivés de celui-ci pour donner des glycérides ou à un alcool pour donner un ester gras).

Par « oxylipine », on entend, au sens de la présente invention, les molécules oxygénées issues de l'oxydation enzymatique ou non enzymatique d'acides gras polyinsaturés. Il peut s'agir en particulier d'octadécanoïdes.

Par « octadécanoïde », on entend, au sens de la présente invention, les molécules oxygénées issues de l'oxydation enzymatique ou non enzymatique des acides gras polyinsaturés à 18 atomes de carbone tels que l'acide linoléique, l'acide alpha-linolénique ou l'acide gamma-linolénique. Il peut s'agir de l'acide 9-hydroxy-10E,12Z-octadécadiénoïque (9-HODE), l'acide 13-hydroxy-9Z,11E-octadécadiénoïque (13-HODE), l'acide 9,10-dihydroxy-12Z-octadécénoïque (9,10-DiHOME), l'acide 12,13-dihydroxy-9Z-octadécénoïque (12,13-DiHOME), l'acide 9,12,13-trihydroxy-10E-octadécénoïque (10-TriHOME), l'acide 9-céto-10E,12Z-octadécadiénoïque (9-OxoODE), l'acide 13-céto-9Z,11E-octadécadiénoïque (13-OxoODE), l'acide 9-hydroxy-10E,12Z,15Z-octadécatriénoïque (9-HOTrE), l'acide 13-hydroxy-9Z,11E,15Z-octadécatriénoïque (13-HOTrE) ou un mélange de ceux-ci.

Par « solvant apolaire », on entend, au sens de la présente invention un solvant lipophile permettant de solubiliser des composés de faible polarité, c'est-à-dire ayant un LogP supérieur ou égal à 1, choisi par exemple parmi l'heptane, l'hexane, le limonène, le chloroforme, le dichlorométhane, le CO₂ supercritique, un mélange CO₂ supercritique / éthanol et les mélanges de ces solvants. On peut citer également les solvants 100% biosourcés comme par exemple EcoXtract^{®} LIPOCOS (Fournisseur Pennakem Europa).

Par « solvant hydrophile », on entend, au sens de la présente invention un solvant polaire choisi par exemple parmi l'eau, l'eau subcritique, les alcools miscibles à l'eau comme par exemple les alcools en C1 à C3, des glycols en C3 à C5, le glycérol, l'acétone, et les mélanges de ceux-ci.

Par « solvant organique non miscible à l'huile », on entend, au sens de la présente invention, un solvant organique qui n'est pas capable de se mélanger, ou seulement partiellement, avec l'huile (c'est-à-dire l'huile provenant d'akènes de *Silybum marianum* (L.) Gaertn.), de sorte que le mélange du solvant organique et de l'huile donne un mélange hétérogène dans lequel il peut être observé au moins deux phases distinctes.

Par « alcool en C1 à C3 », on entend, au sens de la présente invention, un alcool R₂OH dont la chaîne R₂ est une chaîne hydrocarbonée saturée, linéaire ou ramifiée, comprenant 1 à 3 atomes de carbone.

Il pourra s'agir du méthanol, de l'éthanol, du n-propanol ou de l'isopropanol, en particulier du méthanol, de l'éthanol ou de l'isopropanol. De préférence, il s'agira de l'éthanol ou de l'isopropanol. Par « glycol en C3 à C5 », on entend, au sens de la présente invention, une chaîne hydrocarbonée saturée, linéaire ou ramifiée, comprenant 3 à 5 atomes de carbone, ladite chaîne portant 2 fonctions OH. On peut citer par exemple le propylène glycol.

Par « température ambiante », on entend, au sens de la présente invention, une température comprise entre 15 et 40°C, de préférence entre 20 et 30°C, notamment d'environ 25°C.

### Description détaillée de l'invention

L'invention concerne un extrait enrichi en oxylipines issues d'akènes de *Silybum marianum* (L.) Gaertn. pour son utilisation dans le traitement de la séborrhée. La séborrhée peut être la séborrhée de la peau ou bien encore du cuir chevelu.

Les oxylipines sont de préférence des octadécanoïdes tels que le 9-HODE, le 13-HODE, le 9,10-DiHOME, le 12,13-DiHOME, le 10-TriHOME, le 9-OxoODE, le 13-OxoODE, le 9-HOTrE, le 13-HOTrE ou un mélange de ceux-ci. De préférence, les octadécanoïdes sont sélectionnés parmi le 9-HODE, le 13-HODE, le 9,10-DiHOME, le 13-OxoODE, le 13-HOTrE et un mélange de ceux-ci.

La synthèse d'oxylipines dans les akènes de *Silybum marianum* (L.) Gaertn. peut être stimulée avant extraction. Selon un premier mode de réalisation particulier de l'invention, la stimulation peut être effectuée en optimisant les paramètres favorisant les processus d'oxydation, comme la température. Selon un deuxième mode de réalisation particulier de l'invention, la stimulation peut être effectuée par un traitement enzymatique. Selon un troisième mode de réalisation particulier de l'invention, la stimulation peut être effectuée par élicitation, dans le cadre de cultures cellulaires végétales.

L'extrait selon l'invention peut avoir été enrichi en oxylipines par distillation moléculaire, ou par fractionnement, par exemple par des techniques chromatographiques. La synthèse d'oxylipines peut avoir été stimulée dans les akènes de *Silybum marianum* (L.) Gaertn. avant extraction.

Dans un mode de réalisation de la présente invention, l'extrait enrichi en oxylipines peut-être obtenu à partir d'une huile provenant d'akènes de *Silybum marianum* (L.) Gaertn.

L'huile provenant d'akènes de *Silybum marianum* (L.) Gaertn. contient plus particulièrement des acides gras polyinsaturés, et en particulier des acides gras polyinsaturés à 18 atomes de carbone (C18), tel que l'acide linoléique ou l'acide alpha linolénique.

Dans un premier mode de réalisation particulier de l'invention, l'huile provenant d'akènes de *Silybum marianum* (L.) Gaertn. peut être obtenue par pressage des akènes, plus particulièrement à froid, c'est-à-dire sans chauffage, à température ambiante, de préférence suivie d'une étape de filtration.

Dans un deuxième mode de réalisation particulier de l'invention, l'huile provenant d'akènes de *Silybum marianum* (L.) Gaertn. peut être obtenue par extraction des akènes, avec un solvant apolaire, tel que le CO₂ supercritique, avec ou sans ajout d'éthanol comme co-solvant, de préférence suivie d'une étape de filtration.

Dans un troisième mode de réalisation particulier de l'invention, l'huile provenant d'akènes de *Silybum marianum* (L.) Gaertn. peut être obtenue par extraction des akènes avec un solvant hydrophile en présence d'au moins une enzyme (e.g. pectinase), et notamment d'un mélange enzymatique telles que des pectinases par exemple.

Dans un mode de réalisation particulier de l'invention, l'huile provenant d'akènes de *Silybum marianum* (L.) Gaertn. est extraite avantageusement avec une solution aqueuse hydrotropique, de l'eau subcritique, ou un solvant organique non miscible à l'huile éventuellement en mélange avec de l'eau. L'étape d'extraction est avantageusement suivie d'une étape d'enrichissement en oxylipines qui peut être réalisée par distillation moléculaire de l'extrait, ou par fractionnement de l'extrait, par des techniques chromatographiques par exemple.

Ainsi, l'extrait enrichi en oxylipines est avantageusement obtenu par extraction d'une huile provenant d'akènes de *Silybum marianum* (L.) Gaertn. (pour obtenir un extrait d'huile provenant d'akènes de *Silybum marianum* (L.) Gaertn.), suivie d'une étape d'enrichissement en oxylipines (pour obtenir un extrait enrichi en oxylipines d'huile provenant d'akènes de *Silybum marianum* (L.) Gaertn.). L'huile provenant d'akènes de Silybum marianum (L.) Gaertn. peut être obtenue par pressage des akènes, plus particulièrement à froid, de préférence suivie d'une étape de filtration. L'extraction de l'huile peut être réalisée selon le procédé ci-dessous de préparation d'un extrait d'huile.

De préférence, un procédé de préparation d'un extrait d'huile provenant d'akènes de *Silybum marianum* (L.) Gaertn. comprend une étape d'extraction d'une huile provenant d'akènes de *Silybum marianum* (L.) Gaertn. par un solvant d'extraction comprenant, notamment constitué par, un solvant organique non miscible à l'huile provenant d'akènes de *Silybum marianum* (L.) Gaertn. éventuellement en mélange avec de l'eau, une solution aqueuse hydrotropique, ou de l'eau subcritique.

Selon un mode de réalisation particulier, le solvant d'extraction comprend, notamment est constitué par, un solvant organique non miscible à l'huile provenant d'akènes de *Silybum marianum* (L.) Gaertn., éventuellement en mélange avec de l'eau.

Le solvant organique non miscible à l'huile provenant d'akènes de *Silybum marianum* (L.) Gaertn. pourra être notamment un alcool en C1 à C3. Le solvant d'extraction pourra donc être notamment un alcool en C1 à C3 éventuellement en mélange avec de l'eau.

L'alcool en C1 à C3 sera en particulier du méthanol, de l'éthanol, du n-propanol ou de l'isopropanol, en particulier du méthanol, de l'éthanol ou de l'isopropanol. De préférence, il s'agira de l'éthanol ou de l'isopropanol.

Le solvant organique non miscible à l'huile provenant d'akènes de *Silybum marianum* (L.) Gaertn., en particulier un alcool en C1 à C3 tel que le méthanol, l'éthanol ou l'isopropanol, pourra être utilisé en mélange avec de l'eau, notamment dans un rapport volumique solvant organique (e.g. alcool en C1 à C3)/eau de 80/20 à 100/0, notamment de 85/15 à 95/5, en particulier d'environ 90/10.

Le solvant d'extraction pourra notamment être choisi parmi le méthanol, un mélange méthanol/eau, l'éthanol, un mélange éthanol/eau, l'isopropanol et un mélange isopropanol/eau.

Selon un mode de réalisation préféré, le solvant d'extraction sera du méthanol, un mélange éthanol / eau dans un rapport volumique d'environ 90/10 ou un mélange isopropanol / eau dans un rapport volumique d'environ 90/10.

L'étape d'extraction de l'huile provenant d'akènes de *Silybum marianum* (L.) Gaertn. sera effectuée en particulier par mélange de l'huile avec le solvant d'extraction durant 1 h à 12 h et en particulier à une température comprise entre 15°C et 25°C, notamment d'environ 20°C. La quantité de solvant d'extraction utilisé pour effectuer cette extraction sera avantageusement de 0,5 g à 3 g, en particulier de 1 g à 3 g pour 1 g d'huile végétale.

Une phase d'extraction et une phase lipidique seront alors obtenues à la fin de cette extraction. La phase d'extraction sera avantageusement séparée de la phase lipidique et récupérée avant d'être séchée, partiellement ou totalement, notamment sous vide, pour éliminer plus ou moins le solvant d'extraction et obtenir soit un extrait sec si on élimine totalement le solvant, soit un extrait concentré si on élimine partiellement le solvant.

Selon un mode de réalisation selon l'invention, le procédé de préparation d'un extrait d'huile provenant d'akènes de *Silybum marianum* (L.) Gaertn. comprendra les deux étapes successives suivantes :
1. obtention d'une huile provenant d'akènes de *Silybum marianum* (L.) Gaertn., et
2. extraction de l'huile provenant d'akènes de *Silybum marianum* (L.) Gaertn. avec un solvant d'extraction, comprenant, notamment constitué par, une solution aqueuse hydrotropique, de l'eau subcritique ou un solvant organique non miscible à l'huile provenant d'akènes de *Silybum marianum* (L.) Gaertn. éventuellement en mélange avec de l'eau.

Selon un mode de réalisation préféré selon l'invention, le procédé de préparation d'un extrait d'huile provenant d'akènes de *Silybum marianum* (L.) Gaertn. comprendra les étapes successives suivantes :
1. éventuellement obtention d'une huile provenant d'akènes de *Silybum marianum* (L.) Gaertn.,
2. extraction de l'huile provenant d'akènes de *Silybum marianum* (L.) Gaertn. avec un solvant d'extraction, comprenant, notamment constitué par, une solution aqueuse hydrotropique, de l'eau subcritique ou un solvant organique non miscible à l'huile provenant d'akènes de *Silybum marianum* (L.) Gaertn. éventuellement en mélange avec de l'eau, pour donner une phase d'extraction et une phase lipidique,
3. récupération de la phase d'extraction obtenue à l'étape (2), et
4. séchage partiel ou total de la phase d'extraction pour donner un extrait concentré ou un extrait sec.

L'étape (1) pourra être réalisée par pression de la matière première végétale oléagineuse (e.g. les graines, pépins, etc.), notamment par pression à froid, c'est-à-dire sans chauffage, à température ambiante, de préférence suivie d'une étape de filtration.

L'étape (2) sera avantageusement réalisée par pressage des akènes de *Silybum marianum* (L.) Gaertn., plus particulièrement par pression à froid, c'est-à-dire sans chauffage, à température ambiante, de préférence suivie d'une étape de filtration.

L'étape (2) sera avantageusement réalisée avec un solvant d'extraction tel que défini précédemment, et notamment choisi parmi le méthanol, un mélange méthanol/eau, l'éthanol, un mélange éthanol/eau, l'isopropanol, et un mélange isopropanol/eau.

Le solvant organique non miscible à l'huile provenant d'akènes de *Silybum marianum* (L.) Gaertn., en particulier un alcool en C1 à C3 tel que le méthanol, l'éthanol ou l'isopropanol, pourra être utilisé en mélange avec de l'eau, notamment dans un rapport volumique solvant organique (e.g. alcool en C1 à C3)/eau compris de 80/20 à 100/0, notamment compris de 85/15 à 95/5, en particulier d'environ 90/10. Un solvant d'extraction avantageux est un mélange isopropanol / eau dans un rapport volumique d'environ 90/10.

L'étape d'extraction (2) pourra être effectuée par mélange d'huile provenant d'akènes de *Silybum marianum* (L.) Gaertn. avec le solvant d'extraction durant 1 h à 12 h et en particulier à une température de 15°C à 25°c, notamment d'environ 20°C. La quantité de solvant d'extraction utilisé pour effectuer cette extraction sera avantageusement de 0,5 g à 3 g, en particulier de 1 g à 3 g pour 1 g d'huile végétale. Cette étape (2) d'extraction permet d'obtenir à la fin une phase d'extraction d'intérêt et une phase lipidique.

L'étape (3) sera effectuée avantageusement par séparation de la phase d'extraction de la phase lipidique.

L'étape (4) sera avantageusement réalisée sous vide.

Selon un mode de réalisation préféré, l'extrait enrichi en oxylipines selon l'invention est susceptible d'être obtenu par un procédé d'extraction selon l'invention décrit ci-dessus, suivi d'une étape d'enrichissement en oxylipines.

L'extrait enrichi en oxylipines contiendra en particulier au moins 25% en poids, par exemple au moins 30% en poids, par exemple au moins 40% en poids, par exemple au moins 50% en poids, par exemple au moins 60% en poids, par exemple au moins 70% en poids, par exemple au moins 80% en poids, par exemple au moins 90% en poids, d'oxylipines par rapport au poids d'extrait sec. L'extrait enrichi en oxylipines pourra comprendre jusqu'à 100% en poids d'oxylipines.

La présente invention concerne également une composition dermo-cosmétique ou dermatologique comprenant un extrait enrichi en oxylipines d'akènes de *Silybum marianum* (L.) Gaertn. tel que décrit ci-avant, avec au moins un excipient dermo-cosmétiquement ou dermatologiquement acceptable, pour son utilisation dans le traitement de la séborrhée. La séborrhée peut être la séborrhée de la peau ou bien encore du cuir chevelu.

La composition est typiquement sous une forme adaptée à une utilisation par voie topique. Les compositions dermo-cosmétiques ou dermatologiques selon l'invention peuvent se présenter sous les formes qui sont habituellement connues pour une administration topique, c'est-à-dire notamment les lotions, les laits, les émulsions, les sérums, les baumes, les masques, les crèmes, les dispersions, les gels, les mousses, les sprays, les shampooings.

Les compositions dermo-cosmétiques ou dermatologiques selon l'invention, outre l'extrait enrichi en oxylipines et au moins un excipient dermo-cosmétiquement ou dermatologiquement acceptable, peuvent également contenir des agents tensioactifs, des agents complexants, des conservateurs, des antioxydants comme les tocophérols, des agents stabilisants, des émulsifiants, des épaississants, des gélifiants, des humectants, des émollients, des oligo-éléments, des huiles essentielles, des parfums, des colorants, des agents matifiants, des filtres chimiques ou minéraux, des agents hydratants, des eaux thermales, etc..

Les exemples qui suivent, ainsi que les figures, illustrent l'invention sans en limiter la portée.

### Figures

Figure 1 : chromatogrammes UPLC-DAD (234 nm) d'un extrait éthanolique d'huile d'akènes de *Silybum marianum* (L.) Gaertn. et de fractions de celui-ci, A correspondant à la fraction des acides gras libres, B correspondant à la fraction des stérols, C correspondant à la fraction des oxylipines, et D correspondant à l'extrait éthanolique avant fractionnement.
Figure 2a : chromatogramme GC-MS de la fraction 2 (acides gras libres) d'un extrait éthanolique d'huile d'akènes de *Silybum marianum* (L.) Gaertn. (pics majoritaires : (a) acide palmitique, (b) acide oléique, (c) acide linoléique, (d) acide stéarique).
Figure 2b : chromatogramme GC-MS de la fraction 3 (stérols) d'un extrait éthanolique d'akènes de *Silybum marianum* (L.) Gaertn. (pics majoritaires : (a) cholestérol, (b) campestérol, (c) stigmastérol, (d) β-sitostérol, (e) delta-7-stigmastérol).
Figure 2c : chromatogramme GC-MS de la fraction 4 (oxylipines) d'un extrait éthanolique d'akènes de *Silybum marianum* (L.) Gaertn. (pics majoritaires : (a) 9-HODE, (b) 13-HODE).

### Exemples

### Exemple 1 : Extrait lipidique obtenu avec un mélange isopropanol / eau

L'extrait lipidique a été préparé comme suit :
- Pression à froid d'akènes de Chardon-Marie (Silybum marianum(L.) Gaertn.) pour obtenir une huile d'akènes de Chardon-Marie dont la coupe d'acides gras montre une teneur de 55% en acide linoléique ;
- Extraction de l'huile d'akènes de Chardon-Marie par un mélange isopropanol/eau (90/10 v/v) avec 1 gramme du mélange isopropanol / eau par gramme d'huile pendant 2 heures à 20°C ;
- Récupération de la phase isopropanolique, et évaporation du solvant sous vide pour obtenir un extrait lipidique.

L'extrait lipidique a été obtenu avec un rendement massique d'extraction d'environ 3 % et dont les octadécanoïdes principaux sont le 13-HODE, le 9-HODE, le 9,10-DiHOME, le 13-OxoODE et le 13-HOTrE.

### Exemple 2 : Extrait lipidique obtenu avec un mélange éthanol / eau

L'extrait lipidique a été préparé comme suit :
- Pression à froid d'akènes de Chardon-Marie (*Silybum marianum* (L.) Gaertn.) pour obtenir une huile d'akènes de Chardon-Marie dont la coupe d'acides gras montre une teneur de 55% en acide linoléique ;
- Extraction de l'huile d'akènes de Chardon-Marie par un mélange éthanol/eau (90/10 v/v) avec 2 grammes du mélange éthanol / eau par gramme d'huile pendant 2 heures à 20° C ;
- Récupération de la phase éthanolique, et évaporation du solvant sous vide pour obtenir un extrait lipidique.

L'extrait lipidique a été obtenu avec un rendement massique d'extraction d'environ 1 % et dont les octadécanoïdes principaux sont le 13-HODE, le 9-HODE, le 9,10-DiHOME, le 13-OxoODE et le 13-HOTrE.

### Exemple 3 : Fractionnement d'un extrait lipidique d'huile de Silybum marianum obtenu selon l'exemple 2

### ∘ Protocole 1 : Conditions de Fractionnement

- La séparation se fait sur une colonne Chromabond^{™} Flash RS 80 SiOH 40-63 µm (marque Macherey-Nagel^{™}) : volume de colonne de 145 mL, volume de conditionnement de 210 mL.
- L'alimentation en phase mobile se fait à l'aide d'une pompe avec un débit de 10 mL/ min.
- 13 g d'extrait de *Silybum marianum* (L.) Gaertn.obtenu selon l'exemple 2 sont solubilisés dans 40 mL d'heptane.
- La solution obtenue est déposée en tête de colonne puis éluée par 300 mL d'heptane.
- La colonne est ensuite éluée par 800 mL d'un mélange heptane - dichlorométhane (1 : 1) (v/v).
- La colonne est ensuite éluée par 500 mL de dichlorométhane.
- La colonne est ensuite éluée par 500 mL d'un mélange dichlorométhane - méthanol (1 : 1) (v/v).
- La colonne est ensuite éluée par 200 mL de méthanol.

### ∘ Protocole 2 : Conditions analytiques UPLC-DAD

• Colonne : BEH Shield 1.7 µm C18 150*2.1 mm
• Phase mobile :
✔ A : Eau + 0,1% acide formique
✔ B : Acétonitrile + 0,1% acide formique
• Gradient : selon le Tableau 1 ci-dessous

**Tableau 1]**

| **Temps (min)** | **A (%)** | **B (%)** |
|---|---|---|
| 0 | 50 | 50 |
| 1 | 50 | 50 |
| 10 | 0 | 100 |
| 15 | 0 | 100 |
| 16 | 50 | 50 |
| 20 | 50 | 50 |

• Température de la colonne : 50°C
• Débit : 0,4 mL/min
• Détection : 205 nm et 234 nm
• Volume d'injection : 1 µL

### ∘ Protocole 3 : Conditions analytiques CPG-MS

• Colonne : DB-5HT (Agilent Technologies) ; 30m x 0,32 mm x 0,1 µm
• Injection : 320°C ; Mode = Split
• Four : Gradient de température (°C) selon le Tableau 2 ci-dessous

**[Tableau 2]**

| **Temps (min)** | **Température (°C)** |
|---|---|
| 0 | 160 |
| 30 | 370 |
| 40 | 370 |

• Débit Gaz vecteur : 1 mL/min
• Détection : MS Transferline Température = 350°C ; Ion Source Température = 320°C ; MS-EI ; Full Scan Start Mass = 40 ; Full Scan End Mass = 800
• Volume d'injection : 1 µL

### ∘ Résultats :

Les fractions obtenues sont collectées en sortie de colonne et analysées par chromatographie liquide ultra haute pression couplée à un détecteur à barrette de diode (UPLC-DAD), ainsi que par chromatographie en phase gazeuse couplée à un spectromètre de masse (CPG-MS). Elles sont ensuite rassemblées en fonction de leur composition puis mise à sec par évaporation sous vide.

Ce fractionnement permet d'obtenir 4 fractions majoritaires avec, par ordre de polarité croissante les triglycérides, diglycérides, et monoglycérides (Fraction 1 - moins de 1% massique), les acides gras libres (Fraction 2 - 59% massique), les phytostérols (Fraction 3 - 20% massique), puis les oxylipines (Fraction 4 - 20% massique).

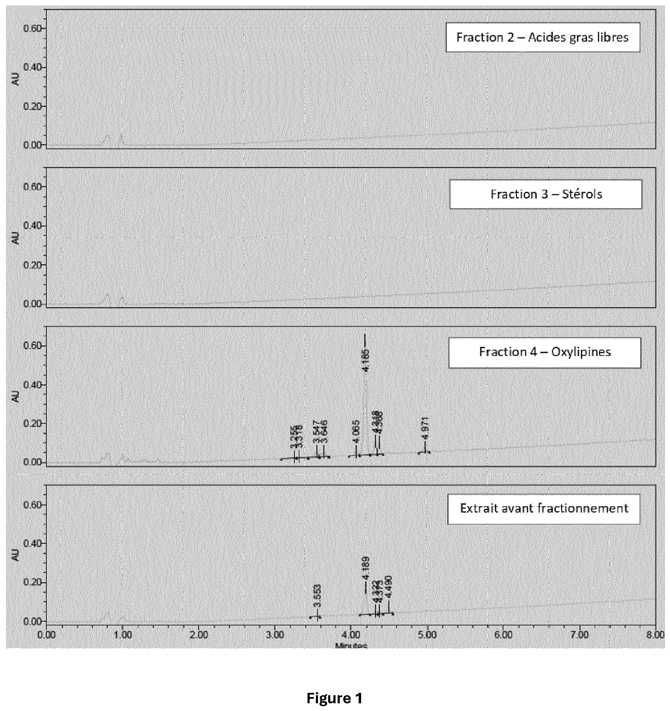

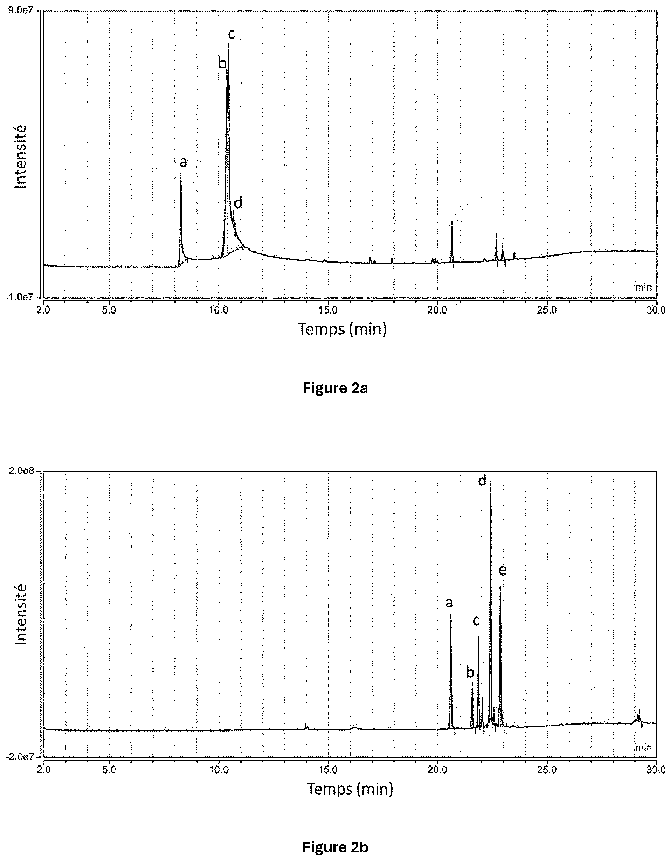

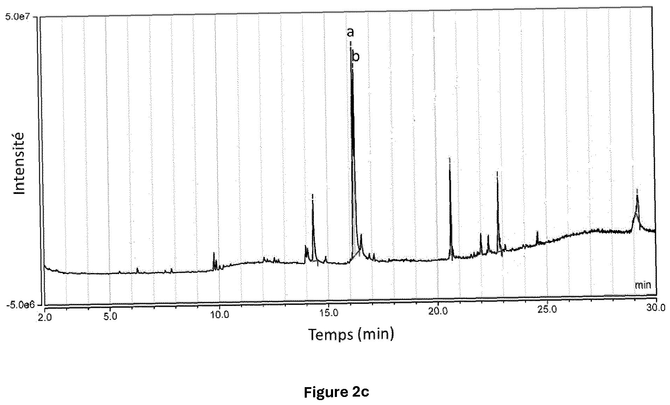

Les chromatogrammes UPLC des fractions 2-4 et de l'extrait avant fractionnement sont présentés sur la Figure 1. Les chromatogrammes GC/MS des fractions 2, 3 et 4 sont présentés sur les Figure 2a, 2b et 2c respectivement.

Ces différentes fractions ont été testées dans l'exemple 4.

### Exemple 4 : Effets d'un extrait de Silybum marianum (L.) Gaertn. sur la lipogenèse induite par l'acide arachidonique dans des sébocytes

Le sébocyte est le type cellulaire majoritaire des glandes sébacées. Ce sont des cellules épithéliales différenciées qui accumulent progressivement des lipides et libèrent leur contenu par dégradation cellulaire et rupture de la membrane cellulaire, ce processus étant connu comme sécrétion holocrine. Pendant la différenciation, les sébocytes accumulent de grandes quantités de lipides dans des vésicules cytoplasmiques sous forme de gouttelettes lipidiques. La différenciation des sébocytes est modulée par plusieurs voies de signalisations, telles que la voie dépendant des PPAR-y ou la voie COX.

### Méthode :

Dans cette étude, un modèle de sébocytes dérivés de cellules souches pluripotentes induites provenant de 2 donneurs différents est utilisé. Ces cellules sont traitées avec de l'acide arachidonique afin d'induire spécifiquement la lipogenèse. L'activité modulatrice d'un extrait lipidique issu d'une huile provenant d'akènes de *Silybum marianum* (L.) Gaertn. est donc évaluée sur la lipogenèse dans les sébocytes.

Cet extrait lipidique issu de l'huile provenant d'akènes de *Silybum marianum* (L.) Gaertn. est préparé selon l'exemple 2. L'extrait est solubilisé dans du DMSO (diméthylsulfoxyde) et est testé à 1µg/mL et 10 µg/mL.

Les sébocytes dérivés des cellules souches pluripotentes induites provenant de 2 donneurs différents sont incubées pendant 3 jours avec une molécule spécifique (PhenoCULT^{®}-NPC PHENOCELL, Grasse, France) pour accélérer leur maturation avant les essais. La lipogenèse des sébocytes est induite par l'acide arachidonique (10 µM) pendant 48 heures en présence ou non d'extrait lipidique d'huile d'akènes de *Silybum marianum* (L.) Gaertn. à tester.

Les résultats sont générés à partir de 2 expérimentations indépendantes, mais faites avec des réplicats, pour obtenir n ≥ 4. Pour les extraits éthanoliques (différentes fractions), les résultats sont en revanche générés à partir d'une seule expérimentation mais avec 6 réplicats

La quantification de la synthèse lipidique est évaluée après coloration Bodipy avec imagerie à haute résolution et normalisée avec la coloration Hoechst (marquage des noyaux).

La comparaison inter-groupes est réalisée par ANOVA à une voie suivie du test de Tukey.

### Résultats :

L'ajout d'acide arachidonique entraine après 48 heures une accumulation importante de lipides totaux dans les sébocytes humains. Ces résultats, à savoir les effets de l'acide arachidonique (AA) sur la production de lipides totaux dans les sébocytes, sont résumés dans le Tableau 3 ci-dessous.

**[Tableau 3]**

| **Lipides totaux** | **Contrôle** | **AA (10µM)** | **AA (10µM) + Produit de référence (1 µM)** |
|---|---|---|---|
| Moyenne ± esm | 80,3 ± 4,7 | 1537,6 ± 593,7 | 144,0 ± 23,1 |

| | | | |
|---|---|---|---|
| AA : acide arachidonique ; esm : erreur standard à la moyenne | | | |

Le composé cannabidiol, produit de référence dans l'inhibition de la lipogénèse, agit en activant les récepteurs ioniques TRPV4 (Olah et al., 2014). Il est testé à 1µM (dissous également dans le DMSO). Il inhibe significativement l'accumulation de lipides. Ce résultat était attendu, il valide le test (Tableau 1).

Le Tableau 4 résume les résultats obtenus avec les différentes fractions obtenues selon l'exemple 3 et testées à 10 µg/mL sur la production de lipides totaux induite par l'acide arachidonique (AA) à 10µM.

**[Tableau 4]**

| **Fractions** | **AA (10µM)** |
|---|---|
| Fraction 2 (acides gras libres) | Pas d'effet |
| Fraction 3 (stérols) | Pas d'effet |
| Fraction 4 (oxylipines)* | Diminution importante |

| | |
|---|---|
| * correspond à un extrait enrichi en oxylipines selon l'invention | |

Seule la fraction 4 avec les oxylipines entraine une diminution de l'accumulation des lipides totaux induite par l'acide arachidonique, indiquant clairement que la fraction d'oxylipines (acides gras oxydés) de l'extrait éthanolique d'akènes de *Silybum marianum* (L.) Gaertn. est capable d'inhiber la lipogénèse induite par l'acide arachidonique.

Le Tableau 5 résume les résultats obtenus avec les extraits lipidiques enrichis en oxylipines d'akènes de *Silybum marianum* (L.) Gaertn. à 1 et 10 µg/mL sur la production de lipides totaux induite par l'acide arachidonique (AA).

**[Tableau 5]**

| **Lipides totaux** | **AA (10µM)** | **AA (10µM) + Extrait selon l'invention (1 µM)** | **AA (10µM) + Extrait selon l'invention (10 µM)** |
|---|---|---|---|
| Moyenne ± esm | 1537,6 ± 593,7 | 600,8 ± 158,7 | 744,5 ± 150,8 |
| Facteur de changement vs AA | - | -2,6 | -2,1 |

| | | | |
|---|---|---|---|
| AA : acide arachidonique ; esm : erreur standard à la moyenne | | | |

L'extrait selon l'invention, aux 2 concentrations testées, diminue significativement (P<0,001 versus le groupe acide arachidonique) les lipides totaux. En effet, le facteur de changement (Fold change en anglais) permet d'apprécier les variations entre différents groupes, une valeur négative indiquant une inhibition et une valeur supérieure à -2 indiquant donc une réduction de plus de 50%.

Les inventeurs mettent clairement en évidence qu'un extrait lipidique d'akènes de *Silybum marianum* (L.) Gaertn. enrichi en oxylipines, notamment les octadécanoïdes principaux (13-HODE, 9-HODE, 9,10-DiHOME, 13-oxoODE, 13-HOTrE) induit une inhibition significative et importante de la lipogénèse induite par l'acide arachidonique dans les sébocytes humains, aux deux concentrations testées.

Ces résultats montrent que les oxylipines issues d'akènes de *Silybum marianum* (L.) Gaertn. sont capables d'inhiber l'accumulation de lipides dans les sébocytes humains et par conséquent réduisent significativement la production de sébum.

Les inventeurs démontrent donc que les oxylipines issues d'akènes de *Silybum marianum* (L.) Gaertn. sont dotées d'une activité anti-séborrhéique.

### Exemple 5 : Fractionnement d'un extrait d'huile de Silybum marianum obtenu selon l'exemple 1

### o Protocole 1 : Conditions de Fractionnement

- La séparation se fait sur une colonne Chromabond^{™} Flash RS 80 SiOH 40-63 µm (marque Macherey-Nagel^{™}), volume de colonne de 145 mL, volume de conditionnement de 210 m L.
- L'alimentation en phase mobile se fait à l'aide d'une pompe avec un débit de 10 mL / min.
- 15 g d'extrait de *Silybum* marianum (L.) Gaertn. obtenu selon l'exemple 1 sont solubilisés dans 40 mL d'heptane.
- La solution obtenue est déposée en tête de colonne puis éluée par 20 mL d'heptane.
- La colonne est ensuite éluée par 400 mL d'heptane.
- La colonne est ensuite éluée par 1000 mL d'un mélange heptane - dichlorométhane (1 : 1) (v/v).
- La colonne est ensuite éluée par 900 mL de dichlorométhane.
- La colonne est ensuite éluée par 250 mL d'un mélange dichlorométhane - méthanol (9 : 1) (v/v).
- La colonne est ensuite éluée par 250 mL d'un mélange dichlorométhane - méthanol (1 : 1) (v/v).

### ∘ Protocole 2 : Conditions analytiques UPLC-DAD

• Colonne : BEH Shield 1.7 µm C18 150*2.1 mm
• Phase mobile :
- A : eau + 0,1% acide formique
- B : acétonitrile + 0,1% acide formique
• Gradient : selon le Tableau 6 ci-dessous

**[Tableau 6]**

| **T (min)** | **A (%)** | **B (%)** |
|---|---|---|
| 0 | 50 | 50 |
| 1 | 50 | 50 |
| 10 | 0 | 100 |
| 15 | 0 | 100 |
| 16 | 50 | 50 |
| 20 | 50 | 50 |

• Température de la colonne : 50°C
• Débit : 0,4 mL/min
• Détection : 205 nm et 234 nm
• Volume d'injection : 1 µL

### ∘ Protocole 3 : Conditions analytiques CPG-MS

• Colonne : DB-5HT (Agilent Technologies) ; 30m x 0,32 mm x 0,1 µm
• Injection : 320°C ; Mode = Split
• Four : Gradient de température (°C) selon le Tableau 7 ci-dessous

**Tableau 7]**

| **T (min)** | **Temp (°C)** |
|---|---|
| 0 | 160 |
| 30 | 370 |
| 40 | 370 |

• Débit Gaz vecteur : 1 mL/min
• Détection : MS Transferline Température = 350°C ; Ion Source Température = 320°C ; MS-El ; Full Scan Start Mass = 40 ; Full Scan End Mass = 800
• Volume d'injection : 1 µL

### o Résultats :

Les fractions obtenues sont collectées en sortie de colonne et analysées par chromatographie liquide ultra haute pression couplée à un détecteur à barrette de diode (UPLC-DAD) ainsi que par chromatographie en phase gazeuse couplée à un spectromètre de masse (CPG-MS). Elles sont ensuite rassemblées en fonction de leur composition puis mise à sec par évaporation sous vide.

Ce fractionnement permet d'obtenir 4 fractions majoritaires avec, par ordre de polarité croissante les triglycérides, diglycérides, et monoglycérides (Fraction 1 - 73% massique), les acides gras libres (Fraction 2 - 5% massique), les phytostérols (Fraction 3 - 8% massique), puis les oxylipines (Fraction 4 - 14% massique).

Ces différentes fractions ont été testées selon la méthode de l'exemple 4.

Le Tableau 8 ci-dessous résume les résultats obtenus avec un extrait lipidique de *Silybum marianum* préparé selon l'exemple 1 et les différentes fractions obtenues selon l'exemple 5 et testées à 10 µg/mL sur la production de lipides totaux induite par l'acide arachidonique (AA) à 10 µM.

**[Tableau 8]**

| **Extrait / Fractions** | **AA (10 µM) + Extrait /fraction (10µM)** |
|---|---|
| | **Facteur de changement vs AA** |
| Extrait sec de *Silybum marianum* (exemple1) | -1,71 |
| Fraction 2 (acides gras libres) | +1,94 |
| Fraction 3 (stérols) | +3,5 |
| Fraction 4 (oxylipines)* | -5,74 |

| | |
|---|---|
| * : Correspond à un extrait enrichi en oxylipines selon l'invention | |

L'extrait sec de *Silybum marianum* non fractionné montre une inhibition significative de l'accumulation des lipides totaux induite par l'acide arachidonique.

Les fractions des acides gras libres et des stérols ne montrent pas d'inhibition de l'induction lipidique. Au contraire ces fractions augmentent fortement la production lipidique des sébocytes stimulés par l'acide arachidonique.

La fraction 4 avec les oxylipines entraine une diminution encore plus marquée que l'extrait de *Silybum marianum* non fractionné de l'accumulation des lipides totaux induite par l'acide arachidonique, indiquant clairement que la fraction d'oxylipines (acides gras oxydés) de l'extrait d'akènes de *Silybum marianum* (L.) Gaertn. est capable d'inhiber la lipogénèse induite par l'acide arachidonique.

### Références bibliographiques

FR2789085
Andreou et al., 2009, Lipids, 44 : 207-215
Blée, 2002, Trends in Plant Science Vol 7 N° 7, 315-321
Calder, 2012, J. Nutr. 142 : 592S-599S
Cuvelier et al., 2004, Ann. Med. Vet. 148, 133-140
Dabbour et al., 2014, Pakistan Journal of Nutrition, 13(2), 67-78
El-Mallah et al., 2003, Grasas y Aceites, 54(4), 397-402
Harrabi et al., 2018, Lipids in Health and Disease, 17, 82
Hermenean et al., 2015, Open Life Sci., 10-225-236
Lagarde, 2011, OCL, vol 18 N°2 mars-Avril, doi.org/10.1051/ocl.2011.0377
Liu et al., 2015, Brain Res. February 9 ; 0 : 220-246
Meddeb et al., 2018, Antioxidants, 7, 95
Oenel et al., 2017, Plant Cell Physiol. 58(5) : 925-933
Olah et al., 2014, J. Clin. Invest. 124(9), 3713-3724
Richardson et al., 2017, J. Agric. Food Chem., 65 : 1941-1951
Rustérucci et al., 1999, J Biol. Chem. Vol 274 N°51: 36446-36455
Van Ginneken et al., 2011, Lipids in Health and Disease, 10, 104
Zarrouk et al., 2019, Current Pharmaceutical Design, 25, 1791-1805
Zhu et al., 2014, Pharmacogn Mag, 10(Sup 1), S92-S99

## Revendications

1. Extrait enrichi en oxylipines d'akènes de *Silybum marianum* (L.) Gaertn. pour son utilisation dans le traitement de la séborrhée.

2. Extrait pour son utilisation selon la revendication 1, **caractérisé en ce que** la séborrhée est celle de la peau ou du cuir chevelu.

3. Extrait pour son utilisation selon la revendication 1 ou 2, **caractérisé en ce que** les oxylipines sont des octadécanoïdes.

4. Extrait pour son utilisation selon la revendication 3, **caractérisé en ce que** les octadécanoïdes sont sélectionnés parmi l'acide 9-hydroxy-10E,12Z-octadécadiénoïque (9-HODE), l'acide 13-hydroxy-9Z,11E-octadécadiénoïque (13-HODE), l'acide 9,10-dihydroxy-12Z-octadécénoïque (9,10-DiHOME), l'acide 13-céto-9Z,11E-octadécadiénoïque (13-OxoODE), l'acide 13-hydroxy-9Z,11E,15Z-octadécatriénoïque (13-HOTrE) et un mélange de ceux-ci.

5. Extrait pour son utilisation selon l'une quelconque des revendications 1 à 4, **caractérisé en ce que** l'extrait enrichi en oxylipines est obtenu par extraction d'une huile provenant d'akènes de *Silybum marianum* (L.) Gaertn., suivie d'une étape d'enrichissement en oxylipines.

6. Extrait pour son utilisation selon la revendication 5, **caractérisé en ce que** l'huile provenant d'akènes de *Silybum marianum* (L.) Gaertn. est obtenue par une étape de pressage, de préférence à froid, des akènes de *Silybum marianum* (L.) Gaertn.

7. Extrait pour son utilisation selon la revendication 5 ou 6, **caractérisé en ce que** l'extraction de l'huile provenant d'akènes de *Silybum marianum* (L.) Gaertn. est réalisée avec un solvant d'extraction comprenant une solution aqueuse hydrotropique, de l'eau subcritique ou un solvant organique non miscible à l'huile provenant d'akènes de Silybum marianum (L.) Gaertn. éventuellement en mélange avec de l'eau.

8. Extrait pour son utilisation selon la revendication 7, **caractérisé en ce que** le solvant d'extraction est un alcool en C1 à C3, éventuellement en mélange avec de l'eau.

9. Extrait pour son utilisation selon l'une quelconque des revendications 1 à 8, **caractérisé en ce que** l'extrait enrichi en oxylipines contient au moins 25% en poids, par exemple au moins 30% en poids, notamment au moins 40% en poids, en particulier au moins 50% en poids, d'oxylipines par rapport au poids d'extrait sec.

10. Composition dermo-cosmétique ou dermatologique comprenant un extrait enrichi en oxylipines d'akènes de *Silybum marianum* (L.) Gaertn. avec au moins un excipient dermo-cosmétiquement ou dermatologiquement acceptable, pour son utilisation dans le traitement de la séborrhée.

11. Composition pour son utilisation selon la revendication 10, **caractérisée en ce que** la séborrhée est celle de la peau ou du cuir chevelu.

12. Composition pour son utilisation selon la revendication 10 ou 11, **caractérisée en ce que** les oxylipines sont des octadécanoïdes.

13. Composition pour son utilisation selon la revendication 12, **caractérisée en ce que** les octadécanoïdes sont sélectionnés parmi le 9-HODE, le 13-HODE, le 9,10-DiHOME, le 13-oxoODE, le 13-HOTrE et leurs mélanges.

14. Composition pour son utilisation selon l'une quelconque des revendications 10 à 13, **caractérisée en ce que** l'extrait enrichi en oxylipines est obtenu par extraction d'une huile provenant d'akènes de *Silybum marianum* (L.) Gaertn., suivie d'une étape d'enrichissement en oxylipines.

15. Composition pour son utilisation selon la revendication 14, **caractérisée en ce que** l'huile provenant d'akènes de *Silybum marianum* (L.) Gaertn. est obtenue par une étape de pressage, de préférence à froid, des akènes de *Silybum marianum* (L.) Gaertn.

16. Composition pour son utilisation selon la revendication 14 ou 15, **caractérisée en ce que** l'extraction de l'huile provenant d'akènes de *Silybum marianum* (L.) Gaertn. est réalisée avec un solvant d'extraction comprenant une solution aqueuse hydrotropique, de l'eau subcritique ou un solvant organique non miscible à l'huile provenant d'akènes de Silybum marianum (L.) Gaertn. éventuellement en mélange avec de l'eau.

17. Composition pour son utilisation selon la revendication 16, **caractérisée en ce que** le solvant d'extraction est un alcool en C1 à C3, éventuellement en mélange avec de l'eau.

18. Composition pour son utilisation selon l'une quelconque des revendications 10 à 17, **caractérisée en ce que** l'extrait enrichi en oxylipines contient au moins 25% en poids, par exemple au moins 30% en poids, notamment au moins 40% en poids, en particulier au moins 50% en poids, d'oxylipines par rapport au poids d'extrait sec.
